# EUROPÄISCHE PATENTSCHRIFT

(11) **EP 1 545 773 B1**
(45) Veröffentlichungstag und Bekanntmachung des Hinweises auf die Patenterteilung: **19.03.2008**
(21) Anmeldenummer: 03766380.4
(22) Anmeldetag: 31.07.2003
(51) Int. Cl.: B01J 31/20, B01J 37/04, C07C 51/12, C07C 51/10, C07C 67/37, C07D 305/12, C07D 303/04

(54) **KATALYSATOR UND VERFAHREN ZUR CARBONYLIERUNG VON OXIRANEN**
CATALYST AND METHOD FOR THE CARBONYLATION OF OXIRANES
CATALYSEUR ET PROCEDE DE CABONYLATION D'OXIRANES

(30) Priorität: 01.08.2002 DE 10235317
(43) Veröffentlichungstag der Anmeldung: 29.06.2005
(73) Patentinhaber: BASF SE, 67056 Ludwigshafen (DE)
(72) Erfinder: LUINSTRA, Gerrit, 68165 Mannheim (DE); MOLNAR, Ferenc, 67346 Speyer (DE); RIEGER, Bernhard, 89295 Oberelchingen (DE); ALLMENDINGER, Markus, 73326 Deggingen (DE)
(74) Vertreter: Isenbruck, Günter
(86) Internationale Anmeldenummer: PCT/EP2003/008479
(87) Internationale Veröffentlichungsnummer: WO 2004/012860

(56) Entgegenhaltungen:
- WO-A-02/12161
- WO-A-03/050154
- GB-A- 1 020 575
- US-A- 3 260 738
- US-A- 4 620 033
- US-A- 6 084 124
- GETZLER Y D Y L ET AL: "SYNTHESIS OF BETA-LACTONES: A HIGHLY ACTIVE AND SELECTIVE CATALYST FOR EPOXIDE CARBONYLATION" JOURNAL OF THE AMERICAN CHEMICAL SOCIETY, AMERICAN CHEMICAL SOCIETY, WASHINGTON, DC, US, Bd. 124, Nr. 7, 2002, Seiten 1174-1175, XP002258049 ISSN: 0002-7863 in der Anmeldung erwähnt
- LEE J T ET AL: "SYNTHESIS OF BETA-LACTONES BY THE REGIOSELECTIVE, COBALT AND LEWIS ACID CATALYZED CARBONYLATION OF SIMPLE AND FUNCTIONALIZED EPOXIDE" JOURNAL OF ORGANIC CHEMISTRY, AMERICAN CHEMICAL SOCIETY. EASTON, US, Bd. 66, Nr. 17, 2001, Seiten 5424-5426, XP002258050 ISSN: 0022-3263 in der Anmeldung erwähnt
- MAHADEVAN V ET AL: "[LEWIS ACID]+[Co(CO)4]- COMPLEXES: A VERSATILE CLASS OF CATALYSTS FOR CARBONYLATIVE RING EXPANSION OF EPOXIDES AND AZIRIDINES" ANGEWANDTE CHEMIE. INTERNATIONAL EDITION, VERLAG CHEMIE. WEINHEIM, DE, Bd. 41, Nr. 15, 2002, Seiten 2781-2784, XP002258051 ISSN: 0570-0833 in der Anmeldung erwähnt
- MOLNAR F ET AL: "MULTISITE CATALYSIS: A MEHANISTIC STUDY OF BETA-LACTONE SYNTHESIS FROM EPOXIDES AND CO-INSIGHTS INTO A DIFFICULT CASE OF HOMOGENEOUS CATALYSIS" CHEMISTRY - A EUROPEAN JOURNAL, VCH PUBLISHERS, US, Bd. 9, Nr. 6, 2003, Seiten 1273-1280, XP002258052 ISSN: 0947-6539 in der Anmeldung erwähnt
- FURUKAWA J ET AL: "COPOLYMERIZATION OF CARBON MONOXIDE WITH ALKYLENE OXIDE" MAKROMOLEKULARE CHEMIE, MACROMOLECULAR CHEMISTRY AND PHYSICS, HUTHIG UND WEPF VERLAG, BASEL, CH, Bd. 89, 1965, Seiten 263-268, XP009001879 ISSN: 0025-116X in der Anmeldung erwähnt
- DATABASE CROSSFIRE BEILSTEIN [Online] Beilstein Institut zur Förderung der Chemischen Wissenschaften, Frankfurt am Main, DE ; Citation Number 5952122; Reaction IDs 4080694, XP002258053 & KOWALCZUK M ET AL: "SYNTHEIS OF NEW GLYCIDYLOXYPROPIOLACTONES" POLISH JOURNAL OF CHEMISTRY, Bd. 55, Nr. 9, 1981, Seiten 1965-1967,

## Beschreibung

Die Erfindung betrifft die Herstellung von Lactonen durch katalytische Carbonylierung von Oxiranen in Gegenwart eines Katalysatorsystems, ein entsprechendes Katalysatorsystem und dessen Verwendung.

Die katalytische Carbonylierung von einfachen und substituierten Oxiranen ist an sich bekannt. Oft sind die Produkte nicht die erwünschten Lactone, oder die Reaktionsführung oder die Ausgangsstoffe lassen eine effiziente Herstellung oder Isolierung von Lactonen nicht zu. Häufig sind dabei die Verbindungen nur durch aufwendige und kostenintensive Synthesen zugänglich.

In der JP-A-09 169 753 ist die Carbonylierung von Epoxiden zu Lactonen in einem Durchlaufreaktor an Co₂CO₈ als Katalysator beschrieben. Die Umsätze betragen nur 30%.
Dies bedeutet, dass zum Erreichen einer hohen Ausbeute und Reinheit des Lactons eine Trennungs- und Rückführungseinrichtung benötigt wird.

GB-A-1,020,575 betrifft ein Verfahren zur Herstellung von Polymeren von β-Lactonen. Kohlenmonoxid und ein 1,2-Epoxid werden zur Bildung eines β-Lactons als Zwischenprodukt umgesetzt. Dabei wird Dicobaltoctacarbonyl als Katalysator eingesetzt. Zudem kann ein Promotor eingesetzt werden, der ausgewählt ist aus Metallhalogeniden wie Kaliumiodid und quarternären Ammoniumhalogeniden wie Tetraethylammoniumbromid. Die Ausbeuten an Lacton betragen jedoch weniger als 10%, die Hauptfraktionen der Produkte sind Polyhydroxypropionester. Zudem wird die Reaktion in einer komplizierten Weise mit mehreren Druckstufen gefahren.

EP-B-0 577 206 betrifft die Carbonylierung von Epoxiden an einem Katalysatorsystem aus einer Cobaltquelle und einer Hydroxy-substituierten Pyridinverbindung, insbesondere 3-Hydroxypyridin oder 4-Hydroxypyridin. Die Carbonylierung wird vorzugsweise in Gegenwart einer Hydroxyverbindung wie Wasser oder Alkoholen durchgeführt. Die Aktivitäten der eingesetzten Katalysatoren sind relativ niedrig, und eine Isolierung der Lactone wird nicht beschrieben. Es wurde ferner beobachtet, dass nach Beenden der Carbonylierung eine Veränderung im Reaktionsgemisch eintritt. Innerhalb von 24 Stunden findet eine Polymerisation des Lactons statt. Hieraus ergibt sich, dass das Lacton in dem Reaktionsgemisch nicht unreaktiv ist. Es ist auch bekannt, dass Lactone unter dem Einfluss von Pyridinen polymerisiert werden können.

Chemistry Letters 1980, Seiten 1549 bis 1552 betrifft die Umsetzung von Epoxiden mit Kohlenmonoxid an einem Rhodiumkomplex als Katalysator. Die Ausbeuten betragen maximal 70%.

In J. Org. Chem. 2001, 66, Seiten 5424 bis 5426 ist die Synthese von β-Lactonen durch Carbonylierung von Epoxiden an Cobalt und Lewissäure-Katalysatoren beschrieben. Als Katalysator wird ein System aus PPNCo(CO)₄ und BF₃ Et₂O) eingesetzt. Die Ausbeuten liegen zwischen 7 und 86%. Die Reaktionsdauer beträgt jedoch 7 bis 24 Stunden, und der Einsatz von großen Katalysatormengen ist notwendig.

In J. Am. Chem. Soc. 124, No. 7, 2002, Seiten 1174 bis 1175 ist die Herstellung von β-Lactonen durch Carbonylierung von Epoxiden beschrieben. Als Katalysator wird ein Gemisch aus einem Salz von Aluminiumsalzen und einem Tetracarbonylcobaltat eingesetzt. Die Handhabung und die Synthese der Aluminiumverbindung sind aufwendig, so dass das Verfahren nicht großtechnisch durchgeführt werden kann.

Aufgabe der Erfindung ist die Bereitstellung eines unaufwendigen und effizienten Verfahrens für die Herstellung von Lactonen durch Carbonylierung von Epoxiden. Eine weitere Aufgabe ist die Bereitstellung eines geeigneten Katalysatorsystems für diese Umsetzung.

Die Aufgabe wird erfindungsgemäß gelöst durch ein Verfahren zur Herstellung von Lactonen durch katalytische Carbonylierung von Oxiranen, dadurch gekennzeichnet, dass ein Katalysatorsystem aus
a) mindestens einer Cobaltverbindung als Komponente A und
b) mindestens einer Metallverbindung der allgemeinen Formel (I) als Komponente B

   MXₓRₙ₋ₓ (I)

   mit der Bedeutung
   - M: Al, Mg oder Zn,
   - R: Wasserstoff oder C₁₋₃₂-Alkyl, C₂₋₂₀-Alkenyl, C₃₋₂₀-Cycloalkyl, C₆₋₁₈-Aryl, C₇₋₂₀-Aralkyl oder C₇₋₂₀-Alkaryl bedeutet, wobei außer am mit M verbundenen Kohlenstoffatom an den Kohlenstoffatomen Substituenten vorliegen können,
   - X: Cl, Br, I, Sulfonat, Oxid, C₁₋₃₂-Alkoxid oder Amid,

   - n: Zahl, die der Wertiglceit von M entspricht,
   - x: Zahl im Bereich von 0 bis n,
   wobei n und x so gewählt sind, dass sich Ladungsneutralität ergibt,
als Katalysator eingesetzt wird.

Die Erfindung wird ferner gelöst durch einen wie vorstehend definierten Katalysator, mit Ausnahme der Kombination Al(C₂H₅)₃/Co(acac)₃.

Das Katalysatorsystem Al(C₂H₅)₃/Co(acac)₃ ist bereits in Die Makromolekulare Chemie 89, 1965, Seiten 263 bis 268 beschrieben. Die Literaturstelle befasst sich mit der Copolymerisation von Kohlenmonoxid mit Alkylenoxiden. Die Bildung von Lactonen wird nicht beschrieben.

Lactone sind wertvolle Verbindungen zur Herstellung von bioabbaubaren Polyestern, s. beispielsweise EP-A-0 688 806. Diese Polyester finden vielfältige Anwendung, beispielsweise als Polyol bei der Polyurethanherstellung oder als Werkstoff.

Es wurde erfindungsgemäß gefunden, dass eine Kombination von Cobaltverbindungen, insbesondere in einem niedrigen Oxidationszustand, und Metallverbindungen für die schonende Carbonylierung von Oxiranen zu Lactonen ein effizientes Katalysatorsystem bilden.

Im erfindungsgemäß eingesetzten Katalysatorsystem liegen vorzugsweise 0,1 bis 1000 mol, besonders bevorzugt 1 bis 100 mol der Komponente B pro mol der Komponente A vor.

Vorzugsweise ist die Komponente A so gewählt, dass unter Umsetzungsbedingungen eine Cobaltcarbonylverbindung vorliegt. Dies bedeutet, dass als Komponente A direkt eine Cobaltcarbonylverbindung eingesetzt werden kann, oder es kann eine Verbindung eingesetzt werden, die unter den Umsatzbedingungen in eine Cobaltcarbonylverbindung umgewandelt wird.

R ist Wasserstoff oder C₁₋₃₂-Alkyl, C₂₀-Alkenyl, C₃₋₂₀-Cycloalkyl, C₆₋₁₈-Aryl, C₇₋₂₀-Aralkyl oder C₇₋₂₀-Alkaryl, wobei außer am mit M verbundenen Kohlenstoffatom an den Kohlenstoffatomen Substituenten vorliegen können. R ist vorzugsweise Wasserstoff oder eine monoanionische Hydrocarbylgruppe, beispielsweise C₁₋₃₂-Alkyl wie Methyl, Ethyl, i- oder n-Propyl, i-, n- oder t-Butyl, n-Pentyl oder n-Hexyl, C₂₋₂₀-Alkenyl wie Propenyl oder Butenyl, C₃₋₂₀-Cycloalkyl wie Cyclopropyl, Cyclobutyl, Cyclopentyl, Cyclopentadienyl oder Cyclohexyl, C₆₋₁₈-Aryl wie Phenyl oder Naphthyl, und C₇₋₂₀-Arylalkyl, z.B. Benzyl (bevorzugte Hydrocarbylgruppe ist Alkyl, besonders bevorzugte Hydrocarbylgruppen sind Methyl oder Ethyl),

X =Cl, Br, J Sulfonat, Oxid, C₁₋₃₂-Alkoxid, Amid; bevorzugte Anionen sind Halogenid oder Alkoxid, besonders bevorzugt sind Chlorid oder C₁₋₁₂-Alkoxid,
wobei n dem Oxidationszustand OZ bzw. der Wertigkeit des Metalls entspricht, x kleiner ist oder den gleichen Wert wie n hat und nicht negativ ist (Für jeden Oxidligand gilt x = x + 1).

Vorzugsweise ist Komponente B AlClₓR₃₋ₓ mit x Zahl von 0 bis 3 und R C₁₋₆-Alkyl. Die Zahlen n und x können dabei ganze oder gebrochene Zahlenwerte darstellen. Gebrochene Zahlenwerte können sich bei einem Gemisch entsprechender Verbindungen ergeben.

Gegebenenfalls kann Komponente A oder B auch einen neutralen Donor L in der Koordinationssphäre binden. Donor L ist im Allgemeinen eine neutrale Verbindung mit Sauerstoff, Stickstoff oder Phosphoratomen, wie Ether, Carbonate, Ketone, Sulfoxide, Amine, Amide, Phosphane, Nitro- oder Nitril- etc. Funktionalitäten. Donor L kann auch ein Olefin oder Aromat sein.

Selbstverständlich können auch Mischungen von mehreren unterschiedlichen Komponenten B und/oder A als Katalysatorsystem benutzt werden.

Besonders bevorzugt ist die Kombination von Dicobaltoctacarbonyl und Trimethylaluminium oder Dicobaltoctacarbonyl und Triethylaluminium oder Dicobaltoctacarbonyl und Tri(sec-Butyl)aluminium, oder Dicobaltoctacarbonyl und Trisisopropoxyaluminium.

Die Carbonylierung wird im Allgemeinen unter erhöhtem Druck und bei erhöhter Temperatur durchgeführt. Allerdings wird auch bei einem Kohlenmonoxiddruck von einer Atmosphäre Produktbildung beobachtet. Der Druck wird im Allgemeinen generiert durch CO-Gas. Dieser Druck kann in bestimmten Fällen auch partial durch ein inertes Medium wie Argon, Stickstoff generiert werden. Die Drücke liegen dabei zwischen 1 und 250 bar, bevorzugt zwischen 10 und 100 bar, besonders bevorzugt zwischen 20 und 60 bar. Die Reaktion kann allgemein bei Temperaturen zwischen -10 und 200 °C durchgeführt werden. Die bevorzugte Temperatur liegt zwischen 20 und 150 °C, besonders bevorzugt zwischen 40 und 110 °C.

Die Carbonylierung von Epoxiden kann sowohl absatzweise als auch in einem kontinuierlichen Verfahren durchgeführt werden. Sie kann sowohl in der Gasphase als auch in einem inerten Reaktionsmedium durchgeführt werden. Dieses Medium ist im Allgemeinen eine Flüssigkeit. Diese sind übliche Lösungsmittel wie Ether, Diglyme, Triglyme, Tetraglyme, Tetrahydrofuran, Dimethoxyethan, Kohlenwasserstoffe wie Hexan, Octan, Isopar, Benzol, Toluol, Xylol, Decalin; chlorierte Kohlenwasserstoffe wie Dichlormethan, Dichlorethan, Dichlorbenzol oder polare Lösungsmittel wie DMF, DMSO, Ester, Nitrile, Nitroverbindungen, Ketone oder sogenannte ionische Flüssigkeiten. Bevorzugte Lösungsmittel sind DME, Diglyme, Dichlormethan. Auch kann das Oxiran als Reaktionsmedium verwendet werden.

Zur weiteren Aktivierung des Katalysatorsystems können Donorliganden hinzugegeben werden, wie Phosphane oder Nitrile. Indem man die Katalysatorkomponenten (z.B. Cobalt und Alkylverbindung) auf ein partikuläres Trägermaterial, z.B. Silica oder Aluminiumoxid aufbringt, ist auch eine lösungsmittelfreie Reaktionsführung im Sinne einer Gasphasencarbonylierung möglich.

Als Oxiranverbindungen sind Ethylenoxid sowie substituierte Epoxide geeignet. Hierbei handelt es sich üblicherweise um solche Verbindungen, die unter die folgende allgemeine Formel (II) fallen:

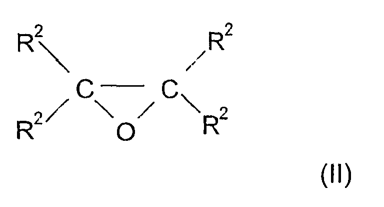

Darin bedeuten die Reste R² unabhängig voneinander Wasserstoff, Halogen, Nitrogruppe - NO₂, Cyanogruppe -CN, Estergruppe -COOR³ oder eine Kohlenwasserstoffgruppe mit 1 bis 32 C-Atomen, die substituiert sein kann. In einer Verbindung (II) können die Reste R² vollständig oder teilweise übereinstimmen oder auch vier unterschiedliche Reste darstellen. R³ kann C₁₋₁₂-Alkyl, Aryl sein.

Bevorzugt wird auf geminal substituierte Epoxide, besonders bevorzugt auf ausschließlich in 1-Position substituierte Epoxide zurückgegriffen.

Geeignete Kohlenwasserstoffgruppen sind beispielsweise C₁₋₃₂-Alkyl wie Methyl, Ethyl, i-oder n-Propyl, i-, n- oder t-Butyl, n-Pentyl oder n-Hexyl, C₂₋₂₀-Alkenyl wie Propenyl oder Butenyl, C₃₋₂₀-Cycloalkyl wie Cyclopropyl, Cyclobutyl, Cyclopentyl oder Cyclohexyl, C₆₋₁₈ -Aryl wie Phenyl oder Naphthyl, und C₇₋₂₀-Arylalkyl, z.B. Benzyl. Dabei können zwei Reste R², falls sie sich an verschiedenen C-Atomen der Epoxygruppe befinden, miteinander verbrückt sein und so eine C₃₋₂₀-Cycloalkylengruppe bilden.

Als Substituenten, mit denen die C₁₋₃₂-Kohlenwasserstoffgruppe wie auch vorstehend R substituiert sein kann, kommen insbesondere folgende Gruppen in Betracht: Halogen, Cyano, Nitro, Thioalkyl, tert.-Amino, Alkoxy, Aryloxy, Arylalkyloxy, Carbonyldioxyalkyl, Carbonyldioxyaryl, Carbonyldioxyarylalkyl, Alkoxycarbonyl, Aryloxycarbonyl, Arylalkyloxycarbonyl, Alkylcarbonyl, Arylcarbonyl, Arylalkylcarbonyl, Alkylsulfinyl, Arylsulfinyl, Arylalkylsulfinyl, Alkylsulfonyl, Arylsulfonyl und Arylalkylsulfonyl.

Bevorzugt verwendet man als Oxiranverbindung Ethylenoxid, Propylenoxid, Butylenoxid (1-Butenoxid, BuO), Cyclopentenoxid, Cyclohexenoxid (CHO), Cycloheptenoxid, 2, 3-Epoxypropylphenylether, Epichlorhydrin, Epibroinhydrin, i-Butenoxid (IBO), Styroloxid oder Acryloxide. Besonders bevorzugt verwendet man Ethylenoxid (EO), Propylenoxid (PO), Butylenoxid oder i-Butenoxid, ganz besonders bevorzugt Ethylenoxid und Propylenoxid oder deren Mischungen.

Die für das erfindungsgemäße Verfahren zu verwendenden Oxiranverbindungen können z.B. über dem Fachmann bekannte Epoxidierungen von endständigen Olefinen gewonnen werden. Verläuft die Epoxidierung stereounspezifisch, so ist eine Racematspaltung vorzunehmen. Methoden zur Racematspaltung, z.B. mittels HPLC-Chromatographie mit chiralem Säulenmaterial, sind dem Fachmann bekannt. Vorteilhafterweise stellt man die Oxiranverbindung ausgehend von einem endständigen Olefin über etablierte stereoselektive Verfahren unmittelbar in enantiomerenreiner oder in optisch angereicherter Form dar. Ein geeignetes Verfahren ist z.B. die so genannte Sharpless-Epoxidierung (s. auch J. Am. Chem. Soc. 1987 (109), S. 5765 ff. und 8120 ff.; sowie "Asymmetric Synthesis", Hrsg. J.D. Morrison, Academic Press, New York, 1985, Band 5, Kapitel 7 und 8).

Des weiteren gelangt man über bei Jacobsen et al., Tetrahedron Lett. 1997, 38, Seiten 773 bis 776; und J. Org. Chem. 1998, 63, Seiten 6776 bis 6777, beschriebene Verfahren, die auch großtechnisch einfach durchzuführen sind, ausgehend von endständigen Olefinen bzw. racemischen terminalen Epoxiden zu optisch angereicherten Oxiranverbindungen (siehe auch Acc. Chem. Res. 2000, 33, Seiten 421 bis 431).

Es ist auch möglich, optisch angereicherte Oxiranverbindungen dadurch herzustellen, dass man zur enantiomerenreinen Oxiranverbindung das Racemat in entsprechender Menge beimengt.

Als Verbindungen mit endständiger Doppelbindung kommen grundsätzlich alle Olefine dieser Verbindungsklasse in Betracht, z.B. Propen, 1-Buten, 1-Penten, 1-Hexen, 1-Hepten oder 1-Octen.

Im Allgemeinen geht man bei der Reaktionsführung so vor, dass zunächst die Cobaltkomplexe (A) und die z.B. Alkylverbindungen (B) einzeln, gleichzeitig oder vorgemischt, gegebenenfalls unter Kühlung, in das Reaktionsgefäß gegeben werden. Auch die Oxiranverbindung kann gegebenenfalls bereits der Lösung/Suspension der Katalysatorkomponenten beigemengt werden, bevor diese in das Reaktionsgefäß überführt wird. Des weiteren kann die Oxiranverbindung auch direkt in das Reaktionsgefäß eingebracht werden. Bevorzugt wird die Carbonylierung unter inerten Bedingungen, d.h. in Abwesenheit von Feuchtigkeit und Luft, durchgeführt.

Abbruch, Trennung und Aufreinigung der Lactone können nach allgemein bekannten Verfahren vorgenommen werden. Beispielsweise lässt sich das Lacton durch Destillation oder Kristallisation auf einfache Weise isolieren.

Mit dem erfindungsgemäßen Verfahren lassen sich ausgehend von enantiomerenreinen Oxiranverbindungen entsprechende 3-Hydroxypropionsäurelactone erhalten. Werden in optisch angereicherter Form vorliegende Oxiranverbindungen eingesetzt, gelangt man zu Lactonen, wobei der Grad an optischer Reinheit unmittelbar mit dem Grad im Oxiran korrespondiert. Ausgehend von so hergestellten Lactonen läßt sich das thermoplastische Eigenschaftsprofil der biologisch abbaubaren Polymerklasse herstellen, dessen Eigenschaften sehr einfach und gezielt für gewünschte Anwendungen eingestellt werden können.

Die Vorteile der Erfindung zeigen sich in der einfachen Fahrweise, sowie in der hohen Aktivität und Produktivität der Carbonylierungskatalyse und der kommerziell erhältlichen Katalysatorkomponenten.

Die Erfindung betrifft auch ein Verfahren zur Herstellung des erfindungsgemäß eingesetzten Katalysators durch Vermischen der Komponenten A und B. Ferner betrifft die Erfindung die Verwendung des Katalysators in Carbonylierungsreaktionen.

### Chemikalien

Die verwendeten Chemikalien stammen von Fluka, Aldrich bzw. Merck und wurden ohne weitere Reinigung eingesetzt. Die Lösemittel wurden trocken über Molsieb erhalten und vor Verwendung jeweils entgast. Die Al-Alkyl Verbindungen wurden als Lösungen in Toluol eingesetzt.

### Analytik

NMR-Spektren wurden auf einem AMX400 von Bruker gemessen. Die IR-Messungen (KBr oder direkt als Lösung) wurden auf den Geräten IFS 113V und IFS 66V von Bruker durchgeführt. Für Online-IR Untersuchungen zur Ermittlung der Reaktionskinetik wurde mit einem React1R^{™} (SiComp^{™} Dippersystem) von Mettler Toledo in einem 250 ml Büchi Reaktor gearbeitet.

### Allgemeine Verfahrensweise:

Zu Dicobaltoctacarbonyl Co₂CO₈ (1 äq.) in Diglyme gab man bei 0 °C unter einer Argonatmosphäre die gewünschte Menge an Epoxid (s.a. Tabellen 1 bis 4). Abschließend wurde eine Verbindung des Typs B (1-6 äq.) zugegeben (s.a. Tabelle 1 bis 4).

Zur Befüllung des Stahlautoklaven (100 oder 250 ml) wurde zunächst evakuiert, die Beschickung erfolgte unter Argon-Gegenstrom. Nach Überführung in den Stahlautoklaven wurde ein Kohlenmonoxiddruck von 10-65 bar eingestellt, und die Carbonylierung wurde über einen vorgegebenen Zeitraum bei Temperaturen von 75 - 105 °C gehalten. Die Carbonylierung wurde durch Druckverminderung auf Umgebungsdruck abgebrochen, die erhaltene Reaktionslösung nach Abkühlen auf 0 °C aus dem Autoklaven entnommen und analysiert. Zur Katalysatorabtrennung kann die erhaltene Lösung in ein Gemisch aus Diethylether/Pentan gegeben werden. Durch Filtration über Kieselgel werden der Katalysator sowie geringste Polymeranteile abgetrennt, eine anschließende destillative Trennung des Filtrats ergibt das Lacton in Reinform.

Die Erfindung wird durch die nachstehenden Beispiele näher erläutert.

### Beispiele

### Beispiel 1:

Dicobaltoctacarbonyl Co₂CO₈ (260 mg) wird in 16 ml Diglyme gelöst, die Lösung wird auf 0 °C gekühlt, und 8 ml Propylenoxid werden zugefügt. Nach Zugabe von 0,77 ml einer 2N Lösung von Me₃Al in Toluol wird die Reaktionslösung unter Ausschluss von Feuchtigkeit und Sauerstoff in einen 100 ml Stahlautoklaven mit Glashülse überführt. Die Carbonylierungsreaktion wird für 5 Stunden unter 60 bar CO bei 75 °C durchgeführt. Der Abbruch der Carbonylierungsreaktion erfolgt durch Druckverminderung auf Umgebungsdruck und. Kühlung auf 0 °C. Die Analyse (¹H- und ¹³C-NMR) einer entnommenen Probe ergibt eine vollständige Carbonylierung des Epoxids und einen Lactonanteil von >95% (Nebenprodukte sind Polyhydroxybutyrat und Aceton).

### Beispiel 2:

In einem 250ml Stahlautoklaven mit IR-Sonde wird unter Argon bei 0 °C Dicobaltoctacarbonyl CO₂CO₈ (780 mg) in 50 ml Diglyme gelöst, und 26 ml Propylenoxid werden zugefügt. Nach Zugabe von 7 ml einer 2N Lösung von Me₃Al in Toluol werden 60 bar CO aufgepresst. Die Carbonylierungsreaktion wird für 2 Stunden unter 60 bar CO bei 95 °C durchgeführt. Der Abbruch der Carbonylierungsreaktion erfolgt durch Druckverminderung auf Umgebungsdruck und Kühlung auf 0 °C. Die Analyse (¹H- und ¹³C-NMR) einer entnommenen Probe ergibt eine vollständige Carbonylierung des Epoxids und einen Lactonanteil von >95% (Nebenprodukte sind Polyhydroxybutyrat und Aceton).

### Beispiel 3:

In einem 250ml Stahlautoklaven mit IR-Sonde wird unter Argon bei 0 °C Dicobaltoctacarbonyl Co₂CO₈ (780 mg) in 50 ml Diglyme gelöst, und 26 ml Propylenoxid werden zugefügt. Nach Zugabe von 7 ml einer 2N Lösung von Me₃Al in Toluol werden 10 bar CO aufgepresst. Die Carbonylierungsreaktion wird für 4 Stunden unter 10 bar CO bei 75 °C durchgeführt. Zum Abbrechen der Reaktion wird der Druck auf Umgebungsdruck abgelassen, und es wird auf 0 °C gekühlt. Die Analyse (¹H- und ¹³C-NMR) einer entnommenen Probe ergibt eine vollständige Carbonylierung des Epoxids und einen Lactonanteil von >95% (Nebenprodukte sind Polyhydroxybutyrat und Aceton).

### Beispiel 4:

Dicobaltoctacarbonyl Co₂CO₈ (130 mg) wird in 8 ml Diglyme gelöst, die Lösung wird auf 0 °C gekühlt, und 7 ml Butyloxiran werden zugefügt. Nach Zugabe von 0,39 ml einer 2N Lösung von Me₃A1 in Toluol wird die Reaktionslösung unter Ausschluss von Feuchtigkeit und Sauerstoff in einen 100 ml Stahlautoklaven mit Glashülse überführt. Die Carbonylierungsreaktion wird für 14 Stunden unter 60 bar CO bei 75 °C durchgeführt. Der Abbruch der Carbonylierungsreaktion erfolgt durch Druckverminderung auf Umgebungsdruck und Kühlung auf 0 °C. Die Analyse (¹H- und ¹³C-NMR) einer entnommenen Probe ergibt eine ca. 70%ige Carbonylierung des Epoxids und einen Lactonanteil im Produkt von > 75%.

### Beispiel 5:

Dicobaltoctacarbonyl Co₂CO₈ (130 mg) und Tetraethylammonium-Cobaltcarbonylat Et₄NCo(CO)₄ (232 mg) werden in 10 ml Diglyme gelöst, die Lösung wird auf 0 °C gekühlt, und 6 ml Propylenoxid werden zugefügt. Nach Zugabe von Aluminiumisopropoxid (i-PrO)₃Al wird die Reaktionslösung unter Ausschluss von Feuchtigkeit und Sauerstoff in einen 100 ml Stahlautoklaven mit Glashülse überführt. Die Carbonylierungsreaktion wird für 16 Stunden unter 60 bar CO bei 75 °C durchgeführt. Der Abbruch der Carbonylierungsreaktion erfolgt durch Druckverminderung auf Umgebungsdruck und Kühlung auf 0 °C. Die Analyse (¹H- und ¹³C-NMR) einer entnommenen Probe ergibt eine nahezu vollständige Carbonylierung des Epoxids und einen Lactonanteil von > 85% (Nebenprodukte sind Polyhydroxybutyrat und Aceton).

In der nachfolgenden Tabelle werden weitere Experimente zusammengefasst, die zeigen, dass hohe Umsätze in kurzer Zeit mit diversen Kombinationen von Cobaltverbindungen und Komponente B erzielt werden.

**Tabelle 1: Variation der Al-Komponente**

| **Nr.** | **Katalysator** | **Epoxid** | **Reaktionsbedingungen** | **Ausbeute** |
|---|---|---|---|---|
| 1 | Co₂CO₈ (läq). Me₃Al (2äq.) | PO (160äq.) | 75 °C/ 60 bar CO/ Diglyme/ 5h | Umsatz 100% Lacton 96% |
| 2 | Co₂CO₈ (1äq.) Et₃Al (2äq.) | PO (160äg.) | 75 °C/ 60 bar CO/ Diglyme/5h | Umsatz 100% Lacton 91 % |
| 3 | Co₂CO₈ (1äq.) (i-Butyl)₃-Al (2äq.) | PO (160äq.) | 75 °C/ 60 bar CO/ Diglyme/ 5h | Umsatz 100% Lacton 87% |
| 4 | Co₂CO₈ (1äq.) Me₃Al (4äq.) | PO (160äq.) | 75 °C/ 60 bar CO/ Diglyme/ 4h | Umsatz 100% Lacton 92% |
| 5 | Co₂CO₈ (1äq.) Me₃Al (6äq.) | PO (160äq.) | 75 °C/ 60 bar CO/ Diglyme/ 3h | Umsatz 100% Lacton 88% |
| 6 | Co₂CO₈(1äq.) EtCl₂Al (2äq.) | PO (160 äq.) | 75 °C/ 60 bar CO/ Diglyme/ 5h | Umsatz 100% Lacton 85% |
| 7 | Co₂CO₈(1äq.) Et₂ClAlClEt₂Al (1äq.) | PO (160äq.) | 75 °C/ 60 bar CO/ Diglyme/5 h | Umsatz 100% Lacton 90% |
| 8 | Co₂CO₈(1äq.) MAO (2äq.) | PO (160äq.) | 75 °C/ 60 bar CO/ Diglyme/ 20h | Umsatz 90% Lacton 80% |
| 9 | Co₂CO₈ (0,5äq.) Et₄NCo(CO)₄ (1äq.) (i-PrO)₃Al(1äq.) | PO (120äq.) | 75 °C/ 60 bar CO/ Diglyme/ 16h | Umsatz 100% Lacton 87% |

Alle Beispiele aus Tabelle 1 wurden in einem 100ml Stahlautoklaven mit Glashülse durchgeführt; Umsätze und Lacton-Anteil wurden mit Hilfe von NMR-Messungen aus einer entnommenen Probe ermittelt; Nebenprodukte sind Polyhydroxybutyrat und in geringen Mengen Aceton.

**Tabelle 2: Druckvariation**

| **Nr.** | **Katalysator** | **Epoxid** | **Reaktionsbedingungen** | **Ausbeute** |
|---|---|---|---|---|
| 10 | Co₂CO₈ (1äq.) Me₃Al (4äq.) | PO (160äq.) | 75 °C/ 80 bar CO/ Diglyme/ 5h | Umsatz 100% Lacton 90% |
| 11 | Co₂CO₈ (1äq.) Me₂Al (4äq.) | PO (160äq.) | 75 °C/ 60 bar CO/ Diglyme/ 5h | Umsatz 100% Lacton 93% |
| 12 | Co₂CO₈ (1äq.) Me₃Al (4äq.) | PO (160äq.) | 75 °C/ 40 bar CO/ Diglyme/ 5h | Umsatz 100% Lacton 92% |
| 13 | Co₂CO₈ (1äq.) Me₃Al (4äq.) | PO (160äg.) | 75 °C/ 20 bar CO/ Diglyme/ 5h | Umsatz 100% Lacton 91% |
| 14 | Co₂CO₈ (1äq.) Me₃Al (4äq.) | PO (160äq.) | 75 °C/ 10 bar CO/ Diglyme/ 5h | Umsatz 100% Lacton 92% |

Alle Beispiele aus Tabelle 2 wurden in einem 250 ml Stahlautoklaven ohne Glashülse, mit IR-Reaktionskontrolle durchgeführt; Umsätze und Lacton-Anteile wurden mit Hife von NMR-Messungen aus einer entnommenen Probe ermittelt; Nebenprodukte sind Polyhydroxybutyrat und in geringen Mengen Aceton.

**Tabelle 3: Temperaturvariation**

| **Nr.** | **Katalysator** | **Epoxid** | **Reaktionsbedingungen** | **Ausbeute** |
|---|---|---|---|---|
| 15 | Co₂CO₈(1äq.) Me₃Al (4äq.) | PO (160 äq.) | 95° C/ 60 bar CO/ Diglyme/ 2h | Umsatz 100% Lacton 92% |
| 16 | Co₂CO₈ (1äq.) Me₃Al (4äq.) | PO (160äq.) | 105 °C/ 60 bar CO/ Diglyme/ 1h | Umsatz 100% Lacton 91%. |

Alle Beispiele aus Tabelle 3 wurden in einem 250ml Stahlautoklaven ohne Glashülse, mit IR-Reaktionskontrolle durchgeführt; Umsätze und Lacton-Anteile wurden mit Hilfe von NMR-Messungen aus einer entnommenen Probe ermittelt; Nebenprodukte sind Polyhydroxybutyrat und in geringen Mengen Aceton.

**Tabelle 4: Variation des Epoxid/Katalysator Verhältnisses**

| **Nr.** | **Katalysator** | **Epoxid** | **Reaktionsbedingungen** | **Ausbeute** |
|---|---|---|---|---|
| 17 | Co₂CO₈ (1äq.) Me₃Al (2äq.) | PO (300äq.) | 75 °C/ 60 bar CO/ Diglyme/ 10 h | Umsatz 100% Lacton 92% |
| 18 | Co₂CO₈(1äq.) Me₃Al (4äq.) | PO (600äq.) | 75 °C/ 60 bar CO/ Diglyme/ 7h | Umsatz 100% Lacton 94% |
| 19 | Co₂CO₈ (1äq.) Me₃Al (4äq.) | PO (1200äq.) | 75 °C/ 60 bar CO/ Diglyme/16 h | Umsatz 80% Lacton 90% |

Alle Beispiele aus Tabelle 4 wurden in einem 100 ml Stahlautoklaven mit Glashülse durchgeführt; Umsätze und Lacton-Anteile wurden mit Hilfe von NMR-Messungen aus einer entnommenen Probe ermittelt; Nebenprodukte sind Polyhydroxybutyrat und in geringen Mengen Aceton.

## Patentansprüche

1. Verfahren zur Herstellung von Lactonen durch katalytische Carbonylierung von Oxiranen, **dadurch gekennzeichnet, dass** ein Katalysatorsystem aus
a) mindestens einer Cobaltverbindung als Komponente A und
b) mindestens einer Metallverbindung der allgemeinen Formel (I) als Komponente B
MXₓRₙ₋ₓ (I)
mit der Bedeutung
M Al, Mg oder Zn,
R Wasserstoff oder C₁₋₃₂-Alkyl, C₂₋₂₀-Alkenyl, C₃₋₂₀-Cycloalkyl, C₆₋₁₈-Aryl, C₇₋₂₀-Aralkyl oder C₇₋₂₀-Alkaryl bedeutet, wobei außer am mit M verbundenen Kohlenstoffatom an den Kohlenstoffatomen Substituenten vorliegen können,
X Cl, Br, I, Sulfonat, Oxid, C₁₋₃₂-Alkoxid oder Amid,
n Zahl, die der Wertigkeit von M entspricht,
x Zahl im Bereich von 0 bis n,
wobei n und x so gewählt sind, dass sich Ladungsneutralität ergibt,
als Katalysator eingesetzt wird.

2. Verfahren nach Anspruch 1, **dadurch gekennzeichnet, dass** die Komponente A so gewählt ist, dass unter Umsetzungsbedingungen eine Cobaltcarbonylverbindung vorliegt.

3. Verfahren nach Anspruch 1 oder 2, **dadurch gekennzeichnet, dass** Komponente B AlClₓR₃₋ₓ ist mit x Zahl von 0 bis 3 und R C₁₋₆-Alkyl.

4. Katalysator, wie er in einem der Ansprüche 1 bis 3 definiert ist, mit Ausnahme der Kombination Al(C₂H₅)₃/Co(acac)₃.

5. Verfahren zur Herstellung von Katalysatoren nach Anspruch 4 durch Vermischen der Komponenten A und B.

6. Verwendung eines Katalysators nach Anspruch 4 in Carbonylierungsreaktionen.

## Claims

1. A process for preparing lactones by catalytic carbonylation of oxiranes, wherein a catalyst system comprising
a) at least one cobalt compound as component A and
b) at least one metal compound of the formula (I) as component B,
MXₓRₙ₋ₓ (I)
where
M is AL, Mg or Zn,
R is hydrogen or C₁₋₃₂-alkyl, C₂₋₂₀-alkenyl, C₃₋₂₀-cycloalkyl, C₆₋₁₈-aryl, C₇₋₂₀-aralkyl or C₇₋₂₀-alkaryl, where substituents may be present on the carbon atoms other than the carbon atom bound to M,
X is Cl, Br, I, sulfonate, oxide, C₁₋₃₂-alkoxide or amide,
n is a number corresponding to the valence of M and
x is in the range from 0 to n,
with n and x being selected so that the compound is uncharged,
is used as catalyst.

2. The process according to claim 1, wherein the component A is selected so that a cobalt carbonyl compound is present under the reaction conditions.

3. The process according to claim 1 or 2, wherein component B is AlClₓR₃₋ₓ where x is from 0 to 3 and R is C₁₋₆-alkyl.

4. A catalyst as defined in any of claims 1 to 3 with the exception of the combination Al (C₂H₅)₃/CO (acac)₃.

5. A process for preparing catalysts according to claim 4 by mixing the components A and B.

6. The use of a catalyst according to claim 4 in carbonylation reactions.

## Revendications

1. Procédé pour la préparation de lactones par carbonylation catalytique d'oxiranes, **caractérisé en ce qu'**un système de catalyseur constitué de :
a) au moins un composé de cobalt en tant que composant (A) et
b) en tant que composant (B), au moins un composé métallique de formule générale (I)
MXₓRₙ₋ₓ (I)
avec les significations suivantes:
M Al, Mg ou Zn,
R hydrogène ou groupe alkyle en C₁₋₃₂, alcényle en C₂₋₂₀, cycloalkyle en C₃₋₂₀, aryle en C₆₋₁₈, aralkyle en C₇₋₂₀ ou alkaryle en C₇₋₂₀, des substituants pouvant être présents sur les atomes de carbone en dehors de l'atome de carbone relié à M,
X Cl, Br, I, groupe sulfonate, oxyde, alcoxyde en C₁₋₃₂ ou amide,
n nombre correspondant à la valence de M,
x nombre dans la plage de 0 à n,
n et x étant choisis de manière à ce qu'une neutralité de charge se produise,
est utilisé en tant que catalyseur.

2. Procédé selon la revendication 1, **caractérisé en ce que** le composant A est choisi de manière à ce qu'un composé de carbonyle de cobalt soit présent dans les conditions réactionnelles.

3. Procédé selon la revendication 1 ou 2, **caractérisé en ce que** le composant B est de formule A1ClₓR₃₋ₓ avec x représentant un nombre de 0 à 3 et R un groupe alkyle en C₁₋₆.

4. Catalyseur, tel qu'il est défini dans l'une quelconque des revendications 1 à 3, à l'exception de la combinaison Al(C₂H₅)₃/Co(acac)₃.

5. Procédé pour la préparation de catalyseurs selon la revendication 4 grâce au mélange des composants A et B.

6. Utilisation d'un catalyseur selon la revendication 4 dans des réactions de carbonylation.
